# EUROPEAN PATENT APPLICATION

(11) **EP 4 295 873 A1**
(43) Date of publication of application: **27.12.2023**
(21) Application number: 22180265.5
(22) Date of filing: 21.06.2022
(51) Int. Cl.: A61M 5/142, A61M 5/145, A61M 5/172

(54) **AN INFUSION CATHETER SYSTEM**

(71) Applicant: National University of Ireland Galway, Galway (IE)
(72) Inventor: Wylie, Robert, Dublin, D15 VYH2 (IE); Connolly, Jack, Wicklow, A63 XK70 (IE); DUFFY, Garry, Galway, H91 DVF7 (IE); Dolan, Eimear, Mayo (IE); Schreiber, Lucien, Galway, H91 Y336 (IE)
(74) Representative: Purdylucey Intellectual Property

(57) **Abstract**

An infusion cannula system suitable for transcutaneous delivery of a treatment fluid to a subject comprises an infusion cannula having a hollow body defining a central lumen having a distal end and a proximal end and a plurality of infusion openings in fluid communication with the central lumen, a treatment fluid reservoir comprising a deflectable membrane in fluid communication with the central lumen of flexible hollow body, a treatment fluid supply conduit in fluid communication with the treatment fluid reservoir, and a deflector configured to deflect the deflectable membrane when actuated to push treatment fluid from the treatment fluid reservoir into the flexible hollow body of the infusion cannula. The treatment fluid reservoir is disposed at a proximal end of the hollow tube and comprises a soft robotic capsule.

## Description

### Field of the Invention

The present invention relates to an infusion catheter system, and in particular a transcutaneous infusion catheter system. The invention also relates to a method of infusing a fluid such as insulin or glucagon into a subject, and in particular a method of transcutaneously infusing a fluid into a subject.

### Background to the Invention

Drug infusion sets provide a way of delivering drug transcutaneously to a subject. The infusion sets include a small short infusion cannula attached to a cannula housing which is attached to a patient's body (for example the arm or abdomen), and a pump and associated tubing to deliver drug from a reservoir to the cannula for infusion. The cannula is implanted transcutaneously into the subject. The cannulas are usually part of a wearable device, such as an infusion set or patch pump device and can be used to deliver drug to the subject continuously or at predetermined time intervals. They are commonly used by subjects with diabetes for the delivery of insulin.

A very common problem associated with the current infusion sets is the foreign body response which leads to the formation of a fibrotic layer or capsule on and around the infusion cannula, causing occlusion. This usually occurs within 4 days of implantation, meaning that the cannula has to be removed and discarded, often prematurely, and a new cannula implanted in a different location. Occlusions can also occur due to improper insertion technique, resulting in kinking of the cannula. A further problem with these devices is that pressure can build up in the infusion cannula due to the fibrotic capsule resulting in release of a bolus volume of drug. In the case of insulin infusion sets, such a bolus release can lead to hypoglycaemia in the subject. Failure of insulin delivery could lead to hyperglycaemia and ketoacidosis.

Some companies are attempting to passively negate this response through an increase in diffusion area by increasing the number of pores in the cannula through which insulin can diffuse. This approach can provide some small enhancements in maintaining insulin diffusion over time. However, this incremental approach is passive, and ultimately limited in its effectiveness in overcoming fibrous obstruction and enabling long-term drug diffusion efficacy.

It is an object of the invention to overcome at least one of the above-referenced problems.

### Summary of the Invention

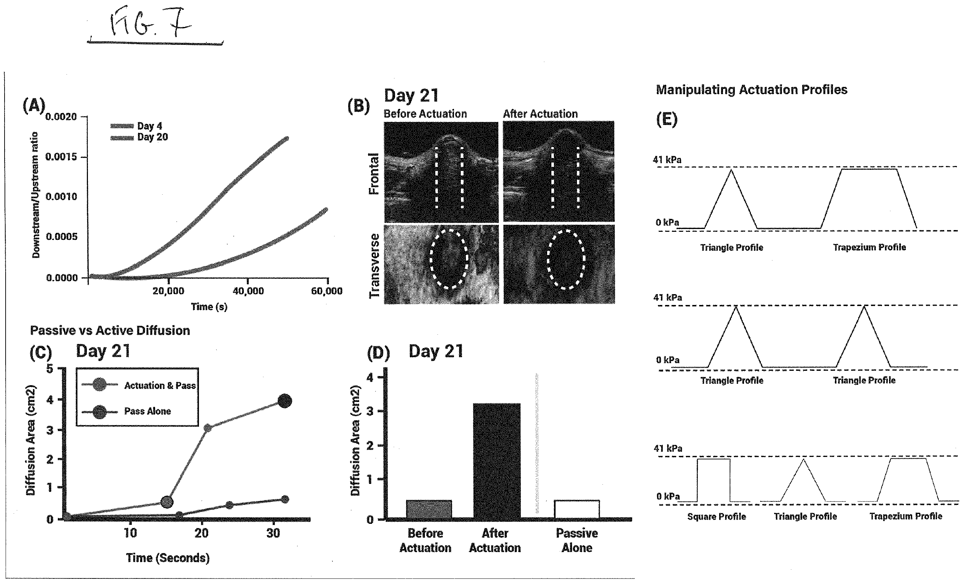

The problems of the prior art are addressed by providing a drug infusion cannula comprising an implantable hollow tube with infusion apertures and drug reservoir fluidically connected to the hollow tube. The drug reservoir is in the form of a soft robotic capsule with a deflectable membrane, and a deflector for the deflectable membrane actuatable to deflect the membrane and push drug from the reservoir into the cannula. The deflector is generally actuatable to deflect the membrane by means of a fluidic (e.g. pneumatic or hydraulic) actuation mechanism, although embodiments of the cannula that comprise different types of actuation mechanisms, such as electrical, magnetic, sonic, mechanical, photothermal or electrothermal actuation mechanisms, are also envisaged. In one preferred embodiment, the deflector is fluidic and is actuatable to exert a fluidic force on the deflectable membrane. In one embodiment, the cannula comprises an actuation chamber containing the drug reservoir. In one embodiment, the cannula comprises a fluid conduit to supply actuation fluid to the actuation chamber to pressurise the deflectable membrane of the drug reservoir and push drug into the cannula upon actuation. In one embodiment, the actuation chamber is implantable and connected to a distal end of the implantable hollow tube. In another embodiment, the cannula is configured such that the reservoir is located outside the body. The provision of a drug reservoir with a deflectable membrane provides a more controllable actuation mechanism allowing more finely tuned and responsive drug delivery from the cannula. In addition, the Applicant has discovered that actuation of the drug reservoir in a sequence of steps, where each step has a different actuation profile (e.g. Figure 7E) provides more effective drug delivery through a fibrotic layer present on the cannula.

In a first aspect, the invention provides an infusion cannula system. The infusion cannula is suitable for transcutaneous delivery of a treatment fluid such as a drug to a subject. The term "treatment fluid" should be understood to include fluids intended for therapeutic or diagnostic purposes. The system generally comprises: an infusion cannula having a hollow body (which is generally flexible) defining a central lumen having a distal end and a proximal end and a plurality of infusion openings in fluid communication with the central lumen;
a treatment fluid reservoir comprising a deflectable membrane and in fluid communication with the central lumen of flexible hollow body;
a treatment fluid supply conduit in fluid communication with the treatment fluid reservoir; and
a deflector configured to be actuated to deflect the deflectable membrane to push treatment fluid from the treatment fluid reservoir into the flexible hollow body of the infusion cannula and out through the infusion openings.

Generally, the treatment fluid reservoir is disposed at a proximal end of the hollow tube. In these embodiments, the treatment fluid reservoir forms part of the implantable part of the cannula. In other embodiments, it is remote to the hollow tube and connected thereto by a fluidic conduit. In these embodiments, the treatment fluid reservoir is generally located outside of the body.

In any embodiment, the treatment fluid reservoir is a resiliently deformable chamber. In any embodiment, the treatment fluid reservoir is a soft robotic capsule. In any embodiment, the deflectable membrane is deformable, typically resiliently deformable.

In any embodiment, the cannula comprises an actuation chamber and the treatment fluid reservoir is disposed within the actuation chamber.

In any embodiment, the deflector is a fluidic deflector.

In any embodiment, the fluidic deflector comprises a deflector chamber, a deflector fluid supply conduit fluidically connected to the deflector chamber, and a first pump operable to pump deflector fluid into the deflector chamber, whereby supply of deflector fluid to the deflector chamber causes the deflector chamber to increase in size and effect deflection of the deflectable membrane.

In any embodiment, the treatment fluid reservoir and deflector chamber are separated by the deflectable membrane.

In any embodiment, the deflector is configured to mechanically deflect the deflectable membrane.

In any embodiment, the deflector is configured to electrically deflect the deflectable membrane.

In any embodiment, the deflector is configured to magnetically deflect the deflectable membrane.

In any embodiment, the deflector is configured to thermally deflect the deflectable membrane (e.g. photothermal actuation or electrothermal actuation).

In any embodiment, the deflectable membrane has a convex configuration.

In any embodiment, the deflectable membrane is deflectable from a convex configuration to a planar or concave configuration.

In any embodiment, the deflectable membrane is deflectable from planar to a concave configuration.

In any embodiment, the treatment fluid supply conduit comprises a lumen disposed within the deflector fluid supply conduit.

In any embodiment, the deflector fluid supply conduit comprises a lumen disposed within the treatment fluid supply conduit.

In any embodiment, the plurality of infusion openings are disposed along a sidewall of the flexible hollow body.

In any embodiment, the plurality of infusion openings are provided by at least a part of the hollow body being formed from a material that is porous to the treatment fluid. Such porous material may be formed by, for example, salt leaching.

In any embodiment, the infusion cannula is dimensioned for transcutaneous implantation to deliver drug sub-dermally or into adipose tissue.

In any embodiment, the infusion cannula has an axial length of 5-20 mm, for example 5-10 mm, 10-20 mm, 10-15 mm or 15-20 mm.

In any embodiment, the infusion cannula has an internal diameter of 0.1 to 1.0 mm or 0.1 to 0.5 mm.

In any embodiment, the system of the invention is wearable.

In any embodiment, the system comprises a treatment fluid storage reservoir fluidically connected to a proximal end of the treatment fluid supply conduit.

In any embodiment, the system comprises a second pump configured to pump treatment fluid from the treatment fluid storage reservoir to the deflectable treatment fluid reservoir upon actuation.

In any embodiment, the system comprises a controller operatively connected to the deflector and operable to control the actuation of the deflector. The controller is configured to control the rate of actuation of the controller (e.g. how often it is actuated). The controller may be configured to control the pressure in the treatment fluid reservoir during deflection, the ramp rate of the pressure, and the actuation profile/waveform (see Figure 7E).

In one embodiment, the controller is pre-programmed to actuate the deflector according to a sequence of steps.

In any embodiment, at least two of the sequential steps comprises a different deflection waveform.

In any embodiment, the controller is configured to perform the sequence of steps a plurality of times per day at predetermined time intervals.

In any embodiment, the system includes a body parameter sensor. The body parameter sensor may be a blood or body fluid sensor. The sensor may be configured to detect a parameter of a body fluid, for example temperature, pH, oxygen, or the level of a component in the body fluid, for example a metabolite such as glucose.

In any embodiment, the sensor is operatively connected to the controller, and in which the controller is configured to actuate the sequence of steps in response to measurements received from the sensor.

In any embodiment, the sensor is a glucose sensor configured to measure glucose in the blood or interstitial fluid of a subject, in which the glucose sensor is operatively connected to the controller, and in which the controller is configured to actuate the sequence of steps in response to blood glucose measurements received from the sensor.

In any embodiment, the sensor is configured for manual actuation of the pumps. This is suitable for diabetes patients who, for example, may wish to actuate the system before a meal to deliver a bolus dose of a fluid such as insulin.

The invention also relates to the use of a system of the invention to deliver a fluid to a subject by cannula infusion, typically transcutaneously.

The invention also relates to a method of diffusing a fluid into a subject that employs an infusion cannula of any preceding Claim, the method comprising the steps of:
Implanting the infusion cannula in tissue of the subject;
advancing a fluid through the fluid supply conduit and into the central lumen of the infusion cannula;
advancing an inflation fluid into the inflatable balloon through the inflation fluid supply conduit to inflate the balloon proximally to distally in the central lumen to force at least some of the fluid within the central lumen out of the infusion cannula through the infusion openings; and
deflating the balloon.

In any embodiment, the infusion cannula is transcutaneously implanted in the subject.

In any embodiment, the fluid comprises a pharmaceutically active agent.

In any embodiment, the pharmaceutically active agent is insulin or glucagon.

In any embodiment, the inflation fluid is a gas such as air or a liquid such as saline

Other aspects and preferred embodiments of the invention are defined and described in the other claims set out below.

### Brief Description of the Figures

**Fig. 1(A)****:** Infusion cannula system of the invention.
**Fig. 1(B)****:** Implantable infusion cannula forming part of the infusion cannula system of the invention and comprising hollow tube and actuation chamber
**Fig 2****:** Infusion cannula with treatment fluid reservoir during treatment fluid filling: (A) prior to filling; (B) during filling; and (C) after filling.
**Fig 3****:** Infusion cannula with deflection chamber and treatment fluid reservoir during actuation (treatment fluid delivery through cannula) : (A) treatment fluid reservoir charged with treatment fluid; (B) deflection chamber is charged with fluid causing the deflectable membrane of the treatment fluid reservoir to deflect pushing treatment fluid into the cannula and out through the holes into the tissue; and (C) after treatment fluid reservoir is re-filled with treatment fluid.

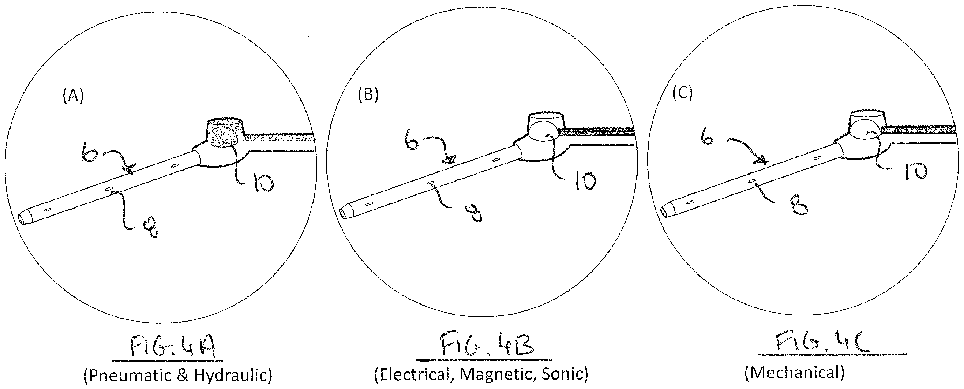

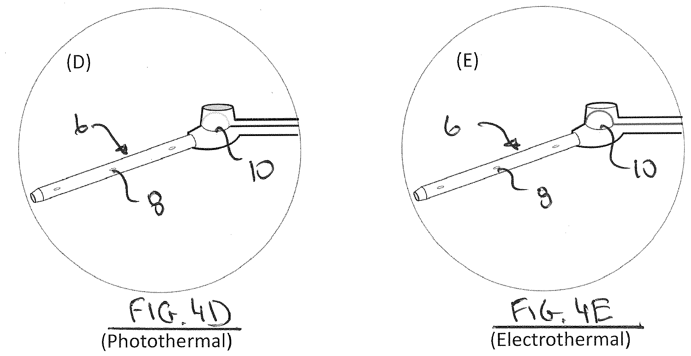

**Fig. 4**: Examples of different embodiments of the invention including (A) a fluidic deflector for the deflectable membrane (B) electrical, magnetic or sonic deflector for the deflectable membrane (C) mechanical deflector for the deflectable membrane (D) photothermal deflector for the deflectable membrane (E) electrothermal deflector for the deflectable membrane.
**Fig. 5****:** illustration of a system of the invention comprising an infusion cannula system, a glucose monitor and a mobile device configured to communicate with the sensor and a control system and which controls the operation of the cannula based on data received from the glucose sensor.

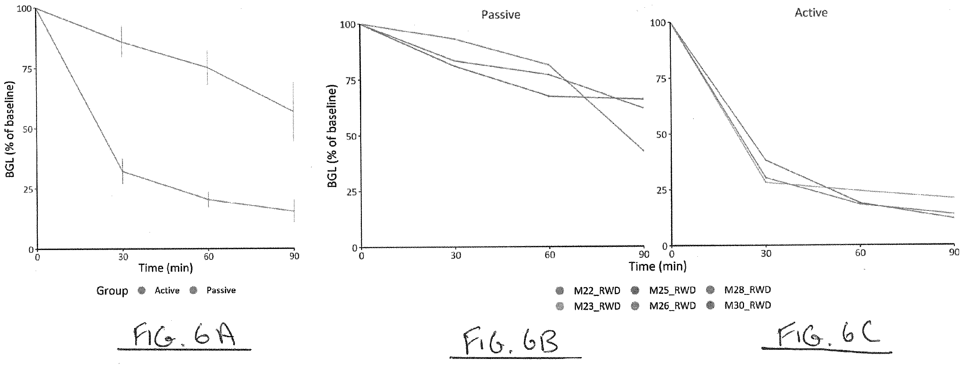

**Fig 6:** (A) Comparison of active vs passive diffusion in terms of drop in Blood glucose level over time. Separate passive (B) and active (C) diffusion blood glucose curves over time.
**Fig 7 (A)****:** Diffusion with respect to time of a drug analogue through the fibrous capsule formed around the device 4 days and 20 days after implantation.
**Fig 7 (B)****:** Diffusion of a drug analogue (shows in red) through the fibrous capsule formed around a device 21 days after subcutaneous implantation in a rat where the drug analogue is concentrated in the reservoir of the device (broken white lines) before actuation and has diffused into the surrounding tissue after actuation.
**Fig 7 (C)****:** Quantification of the diffusion area (cm2) with respect to time for passive diffusion along (blue line) and actuation-assisted diffusion (red line).
**Fig 7 (D)****:** Quantification of the diffusion area (cm2) of the drug analogue before actuation, after actuation and for passive diffusion alone, where bars correspond to matching colours in graph in part C.
**Fig 7 (E)****:** Modifying the waveform of the actuation regime gives a high level of control of drug delivery profiles.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or openended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, age, poisoning or nutritional deficiencies.

As used herein, the term "treatment" or "treating" refers to an intervention (e.g. the administration of an agent to a subject) which cures, ameliorates or lessens the symptoms of a disease or removes (or lessens the impact of) its cause(s) (for example, the increase in levels of a tight junction protein). In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

As used herein, an effective amount or a therapeutically effective amount of an agent defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure. Improvement may be observed in biological / molecular markers, clinical or observational improvements. In a preferred embodiment, the methods of the invention are applicable to humans, large racing animals (horses, camels, dogs), and domestic companion animals (cats and dogs).

In the context of treatment and effective amounts as defined above, the term subject (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, camels, bison, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human. As used herein, the term "equine" refers to mammals of the family Equidae, which includes horses, donkeys, asses, kiang and zebra.

"Infusion cannula" refers to an implantable cannula having a hollow body with infusion openings configured to allow liquid within the hollow body pass into surrounding tissue in which the cannula is implanted. The infusion cannula is generally a small infusion cannula (e.g having an axial length of less than 30 or 25 mm and/or an internal diameter of less than 1.5, 1.0, 0.75, 0.5 or 0.3 mm) such as a transcutaneous infusion cannula which generally has a length of 6-20 mm and an internal diameter of 0.15 to 1.0 mm. In one embodiment, the cannula is a 25G cannula. Small infusion cannula's are more prone to blockage due to fibrotic encapsulation compared with larger cannula's such as central line cannula's. The cannula is generally flexible. The cannula may be formed of any suitable material, the details of which will be known to a person skilled in the art. Examples of materials include silicone, polyurethane, polyethylene, polyvinylchloride, PTFE and nylon. In one embodiment, the infusion cannula is not an intravenous cannula. The cannula generally has an open distal end dimensioned to allow a delivery needle be advanced axially through the lumen of the cannula during implantation of the infusion cannula.

"Transcutaneous" as applied to an infusion cannula means an infusion cannula configured for administration through the skin and implantation under the skin or in the adipose layer under the skin. Transcutaneous infusion cannulas are often designed for self-implantation by a patient using an applicator (e.g. Medtronic MINIMED^{™} infusion set), and do not require a physician for implantation.

"Infusion openings" refer to apertures that allow infusion of fluid from inside the cannula to the surrounding tissue. The apertures generally are small pores formed in the cannula side wall that are generally co-extensive with at least a distal end, and generally the full length, of the balloon. The apertures are generally disposed on a side of the cannula, and optionally also at a distal tip of the cannula. In the embodiments shown, the cannula has two series of four infusion openings disposed axially on opposite sides of the cannula side wall. It will be appreciated that the infusion openings may be provided in any number or arrangement. The openings may have a size ranging from 5 to 300 microns. The pores may be configured so that the cannula has a porosity of 5 to 95% (i.e the % area of the cannula sidewall that is open due to the pores). The infusion openings may also be of any shape, including round holes or elongated slits. The infusion openings may have the same internal diameter from inside to outside, or may be conical having a larger inlet than outlet or *vica versa.* In some embodiments, the cannula may be formed from a material that is itself porous, where the pores provide the infusion openings. Cannula's that are porous may be formed by porogen leaching techniques (Jeffrey A. Coffer et al PSS Vol 202, Issue 8 June 2005). Porous cannulas may be made from Silicone, Pebax, Polyurethane, Teflon, PTFE, Nylon elastomers, Dacron and other thermoplastic elastomers.

"Treatment fluid reservoir" refers to a chamber having a deflectable membrane, an inlet to fluidically connect with a treatment fluid supply conduit, and an outlet to fluidically connect with the lumen of the hollow tube of the cannula. The reservoir is generally disposed at a proximal end of the hollow tube and is part of the implantable infusion cannula. In other embodiment, the reservoir (or actuation chamber containing the reservoir) is remote to the hollow tube and connected thereto by a length of tubing. The chamber generally takes the form of a soft robotic capsule. The deflectable membrane may be convex and be adjustable into a planar or concave configuration.

"Deflector" refers to an apparatus configured to deflect the deflectable membrane of the treatment fluid reservoir upon actuation. Generally, the deflector is contained with the actuation chamber. In the embodiments shown in the drawings, the deflector is fluidic, and comprises an actuation chamber which contains the treatment fluid reservoir and that is fluidically connected to an actuation fluid supply conduit. Pumping actuation fluid, generally a liquid such as saline, into the chamber causes the deflectable membrane of the treatment fluid reservoir to deflect and push treatment fluid into the hollow tube of the infusion cannula. The deflectable membrane may be convex, for example dome shaped. The deflectable membrane may be deflectable from a convex shape to a planar or concave shape (or a less convex shape). In another embodiment, the actuation chamber may include an actuation fluid reservoir that is fluidically connected to the actuation fluid supply conduit and is configured to bear against the deflectable membrane when actuation fluid is pumped into the actuation fluid reservoir. In other embodiment, the deflector may an electrical or electro-mechanical apparatus configured to deflect the deflectable membrane when actuated. In other embodiments, the deflector may form part of the deflectable membrane and be actuatable by electrical, magnetic, sonic, or thermal (photothermal or electrothermal)means to cause the membrane to deflect. In one embodiment, the deflectable membrane comprises a dielectric elastomer or a piezoelectric material.

"Fluid" refers to an air, gas or liquid or mixture thereof. When the deflector is fluidic, the fluid is generally a liquid such as saline, but may also be air. The treatment fluid is generally a drug, for example a drug that is self-administered such as insulin, or a diagnostic reagent. Other types of drug that may be self-administered are antiinflammatory, anti-microbial, pain medicaments, and chemotherapeutic drugs. The drug is generally fluid and may be a solution or suspension.

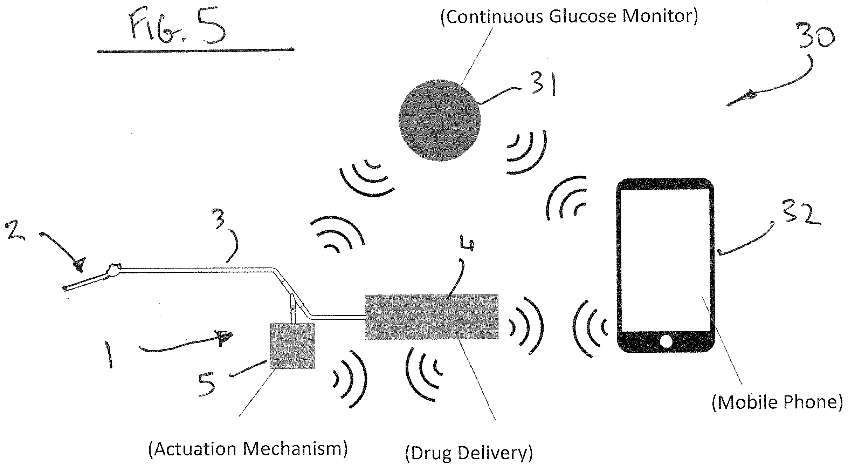

"Controller" refers to a device operatively connected to the fluid pump(s) and configured to control the actuation of the pump(s). The system generally includes a pump for the actuation fluid supply conduit (actuation pump) and a separate pump for the treatment fluid supply conduit (treatment pump). The Controller may be configured to actuate the pump to re-fill the treatment fluid reservoir after it has been emptied. The Controller may be configured to actuate the actuation pump to push treatment fluid into the hollow tube. The controller may be configured to control one or more of the following actuation parameters: time of actuation; frequency of actuation; ramp speed of actuation; max pressure (Pmax) of actuation; time at pmax. In one embodiment the controller is configured to actuate the actuation pump to perform a sequence of actuation steps. Typically at least two (and preferably all) of the actuation steps have a different actuation profile. The actuation profile of the actuation steps may differ in the actuation waveform (See Figure 5E), which may be selected from square, triangular, trapezium. The controller may be programmable to actuate the system at predetermined times, for example once a day, several times a day, or as required by the user. In addition, the parameters of actuation may be controlled by the controller, for example the volume of drug to be delivered, the drug infusion time, and pressure of drug delivery, and the time period between balloon inflation and balloon deflation. The controller generally comprises a processor operably connected to the controller. The processor may be pre-programmable. The controller may include a graphic display. The controller may include a user interface. The controller and processor may be contained within the same housing. The controller is generally wearable. The controller may include a wireless communications module configured to communicate with a separate processor. The separate processor may be a mobile communications device comprising software comprising instructions for the processor to control the operation of the controller. The software may be downloadable software (e.g. a mobile communications "app"). The software may be configured to graphically display data relating to the operation of the system on a screen of the controller or a mobile communications device.

"Sensor" refers to a device that can measure a body parameter, for example, pH, or the presence or abundance of an element (such as a drug or metabolite) in the body and especially in a body fluid such as blood or interstitial fluid. Examples include sensors for detecting the level of a metabolite in a body fluid such as blood

or interstitial fluid, for example a glucose sensor. The system of the invention may include a sensor, or be configured for operative connection with a sensor. The controller forming part of the system of the invention may be operatively connected to the sensor and actuate the system in response to data received from the sensor. The controller may be configured to actuate the system of the invention when the level of a metabolite reaches a threshold level. For example, when the system of the invention is for infusing insulin, the system may be configured to actuate in response to data from the sensor indicating that the glucose levels have reached a defined threshold level.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

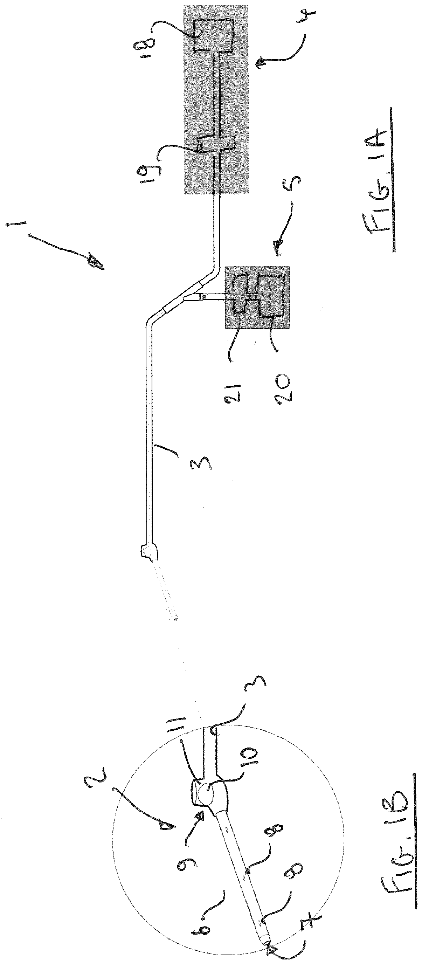

Referring to the drawings and initially to Figures 1 to 3, there is illustrated an infusion cannula system of the invention indicated generally by the reference numeral 1 and comprising an infusion cannula 2, fluid supply conduit 3, drug supply module 4 and actuation fluid supply module 5.

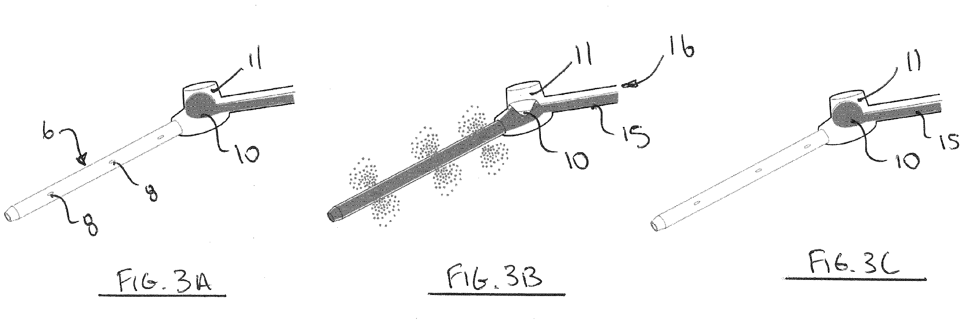

The infusion cannula 2 comprises a flexible tube 6 with an internal lumen 7 and apertures 8, and an actuation chamber 9 comprising a drug reservoir 10 and a deflection chamber11 separated by a deflectable membrane 13 . In this embodiment, the infusion cannula 1 has a length of about 3 cm and a width of about 0.3 cm. It is dimensioned to be implanted under the skin of a patient and to deliver drug subcutaneously upon actuation. Thus, the drug reservoir 10 is fluidically connected to the internal lumen 7 of the cannula 1 and the deflectable membrane 13 is configured to deflect from a convex dome shape shown in Figure 3A to a concave shape shown in Figure 3B to push drug from the drug reservoir 10 into the cannula 1 and out through the apertures 8 of the cannula.

The fluid supply conduit 3 connects the infusion cannula which is under the skin with the other parts of the system which are disposed outside the body. The conduit 3 comprises a treatment fluid supply conduit 15 that fluidically connects the drug reservoir 10 with the drug supply module 4 and an actuation fluid supply conduit 16 that fluidically connects the actuation volume 11 of the actuation chamber 9 with the actuation fluid supply module 5. The drug supply module 4 comprises a storage chamber 18 for drug and a pump 19 configured to pump drug from the storage chamber 18 to the drug reservoir 10 upon actuation. The actuation fluid supply module 5 comprises a storage chamber 20 for actuation fluid and a pump 21 configured to pump drug from the storage chamber 20 to the actuation volume 11 of the actuation chamber 9 upon actuation.

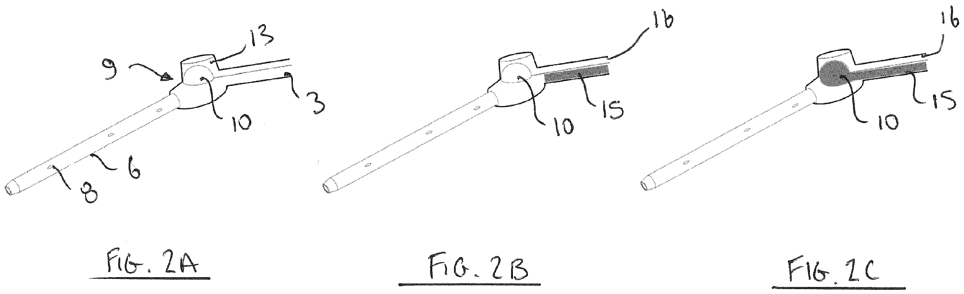

In use, and referring to Figs 2 and 3, the pump 19 is actuated to pump drug from the storage chamber 18 to the drug reservoir 10 to charge the reservoir with drug as shown in Figure 2C. The pump 21 is then actuated to pump actuation fluid into the volume 11 of the actuation chamber, which pressurises the chamber and causes deflection of the deflectable membrane 13 from the dome shape shown in Figure 3A to the concave shape of Figure 3B, thereby pushing drug into the cannula 1 and out through the apertures 8.

Referring to Figure 5, there is illustrated a system of the invention indicated generally by the reference numeral 30 and in which parts identified with reference to the previous embodiments are assigned the same reference numerals. The system comprises the infusion cannula system 1 described above, a wearable glucose sensor 31 configured to measure blood glucose levels of a subject, and a controller 32 configured to receive data from the sensor wirelessly and then actuate the cannula system 1 based on the received data. In this embodiment, the controller 32 is provided by a mobile phone with controlling software running on the device. The software may be downloadable software (e.g. an "app"). The software may comprise program instructions to instruct the device to communicate wirelessly with the sensor and the two pumps 198, 21 of the cannula system 11', to actuate the pumps according to a pre-programmed sequence of steps. The sequence chosen by the controller may be determined by data received from the sensor. The controller may control the actuation of the infusion cannula, for example when it actuates (e.g. at what time of the day, how many times per day) and how it actuates (e.g. actuation profile). The controller may be wearable. The system of the invention may be wearable. For example, the controller may be configured to actuate the infusion cannula in a sequence of actuation steps, for example 2 to 100 sequential actuation steps. The sequence of steps may be performed over a period of time, for example, 1 to 60 minutes. At least two of the actuation steps (or most or all) may have a different actuation profile, e.g. square, triangular, trapezium shaped.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. An infusion cannula system suitable for transcutaneous delivery of a treatment fluid to a subject, the system comprising:
an infusion cannula having a hollow body defining a central lumen having a distal end and a proximal end and a plurality of infusion openings in fluid communication with the central lumen;
a treatment fluid reservoir comprising a deflectable membrane in fluid communication with the central lumen of flexible hollow body;
a treatment fluid supply conduit in fluid communication with the treatment fluid reservoir; and
a deflector configured to deflect the deflectable membrane when actuated to push treatment fluid from the treatment fluid reservoir into the flexible hollow body of the infusion cannula and out through the infusion openings.

2. An infusion cannula according to Claim 1, in which the treatment fluid reservoir is disposed at a proximal end of the hollow tube.

3. An infusion cannula according to Claim 1 or 2, in which the treatment fluid reservoir is a soft robotic capsule.

4. An infusion cannula according to any preceding Claim, in which the cannula comprises an actuation chamber and the treatment fluid reservoir is disposed within the actuation chamber.

5. An infusion cannula according to Claim 4, in which the deflector is a fluidic deflector.

6. An infusion cannula according to Claim 5, in which the fluidic deflector comprises:
a deflector chamber that is disposed within the actuation chamber and separated from the treatment fluid reservoir by the deflectable membrane;
deflector fluid supply conduit fluidically connected to the deflector chamber; and
a first pump operable to pump deflector fluid into the deflector chamber,
whereby supply of deflector fluid to the deflector chamber causes the deflector chamber to increase in size and effect deflection of the deflectable membrane.

7. An infusion cannula according to any of Claims 1 to 4, in which the deflector is configured to mechanically, acoustically, magnetically or thermally deflect the deflectable membrane.

8. An infusion cannula according to any preceding Claim, in which the deflectable membrane has a convex configuration prior to deflection.

9. An infusion cannula according to any preceding Claim, in which the infusion cannula has an axial length of 5-20 mm.

10. An infusion cannula system comprising an infusion cannula according to any preceding Claim and a controller operatively connected to the deflector and operable to control the actuation of the deflector.

11. An infusion cannula system according to Claim 10, in which the controller is configured to control a parameter of the actuation of the deflector selected from the rate of actuation of the deflector, the pressure in the treatment fluid reservoir during actuation of the deflector, the ramp rate of the pressure in the treatment fluid reservoir during actuation, and the actuation profile/waveform.

12. An infusion cannula system according to Claim 10 or 11, in which the controller is pre-programmed to actuate the deflector according to a sequence of steps in which at least two of the sequential steps comprises a different deflection waveform.

13. An infusion cannula system according to any of Claims 10 to 12, in which the system includes a body parameter sensor.

14. An infusion cannula system according to Claim 13, in which the body parameter sensor is operatively connected to the controller, and in which the controller is configured to actuate the deflector in response to measurements received from the sensor.

15. An infusion cannula system according to any of Claims 10 to 14, in which the deflector is a fluidic deflector comprising a pump, in which the controller is configured to actuate the deflector in response to measurements received from the sensor.
